# EUROPEAN PATENT APPLICATION

(11) **EP 4 209 172 A1**
(43) Date of publication of application: **12.07.2023**
(21) Application number: 22757470.4
(22) Date of filing: 07.07.2022
(51) Int. Cl.: A61B 5/00, A61B 5/0537, A01K 15/04, A01K 1/06, A01K 29/00, A01K 1/03, A01K 5/01, A01K 15/02

(54) **DEVICE FOR MEASURING BIOMETRIC INFORMATION OF COMPANION ANIMAL**

(30) Priority: 23.11.2021 KR 20210162054; 10.03.2022 KR 20220029956
(71) Applicant: The LittleCat.Co.,Ltd., Chuncheon-si, Gangwon-do 24465 (KR)
(72) Inventor: KIM, Daeyong, Seoul 07532 (KR); KIM, Yujeong, Anyang-si, Gyeonggi-do 14017 (KR)
(74) Representative: Ter Meer Steinmeister & Partner
(86) International application number: PCT/KR2022/009829
(87) International publication number: WO 2023/096051

(57) **Abstract**

The present disclosure relates to a technology of a system for measuring biometric information of a companion animal, the system including a body unit having a plate unit on which a companion animal steps; an induction unit installed on a side of the plate unit and inducing a companion animal to maintain a posture in a pre-defined target area for a predetermined time; a plurality of electrode units installed on the plate unit; and a processor unit measuring biometric information of the companion animal on the basis of output signals of the electrodes. According to the present disclosure described above, there is an effect that since it is possible to measure exact biometric information of a companion animal, it is possible to check the health condition of a companion animal and enable the companion animal to lead a healthy life.

## Description

### Technical Field

The present disclosure relates to a technology of measuring biometric information of a companion animal and, in more detail, a technology enabling exact measurement of biometric information by inducing a companion animal to maintain a fixed posture in a target area over a predetermined time.

### Background Art

As the number of animal lovers who own companion animals increases, the demands for devices or tools for managing the health of companion animals have been increased.

For example, a body composition measuring device and so on is used as a health management device for companion animals. As a device for measuring the body composition of the companion animals, a device for measuring bio-impedance and calculating data on the amount or the ratio of body fat, muscle, body water, or bone as body composition data based on this has been researched. The most common biometric device for companion animals is to obtain body composition data by contacting impedance measuring electrodes to each of the four soles of the feet and then measuring the impedance of the animal's torso body.

However, unlike humans, when measuring the body composition of the animals such as a dog or a cat, it is difficult to accurately measure the body composition of the animals because of the tendency of the animal to keep moving. Accordingly, in order to accurately measure the body composition of the animals such as a dog or a cat, a method capable of restricting the movement of the animals is required.

In addition, prior to restricting the movement of the animals, it is very difficult to induce the companion animals to stand on the measurement device when measuring the body composition of the companion animals. Therefore, a method for guiding the companion animals to the measurement position is more important.

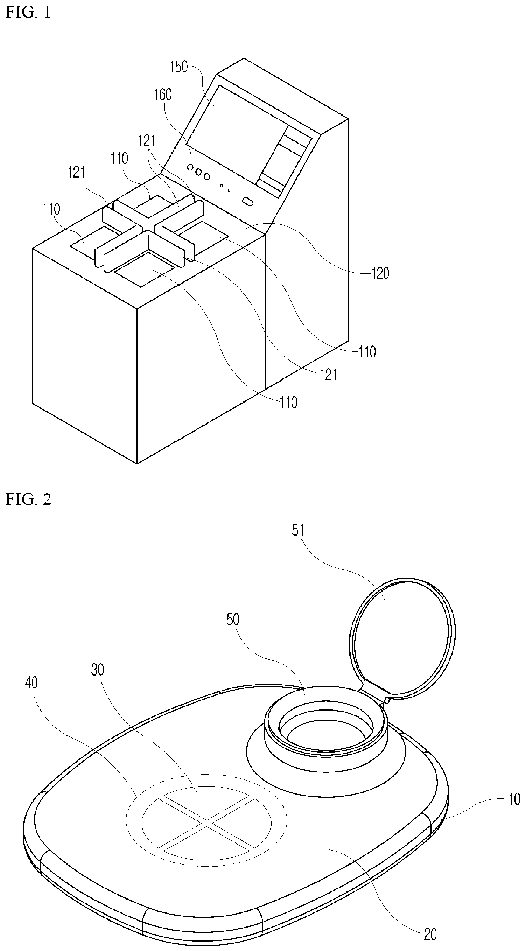

Accordingly, as shown in FIG. 1, an apparatus capable of more accurately measuring the body composition of the animal by restricting the movement of the animal when measuring the body composition of the animal such as a dog or a cat have been proposed.

Referring to FIG. 1, the animal body composition analyzing apparatus 100 includes movement prevention walls 121 for preventing movement of the animal's feet on the opposite side between the electrode body 110 and the other electrode body 110. When the four feet of the animal each come into contact with the plurality of electrode bodies 110 in a state that the electrode body, which is in contact with the animal's feet, is installed to be spaced apart from the ground at a certain height, the animal may move out of the electrode bodies 110 by moving its legs while being afraid and hesitant. Accordingly, by forming the movement prevention walls 121 to prevent movement of the animal's feet on the opposite side between the electrode body 110 and the other electrode body 110, the animal's feet do not come out of the electrode bodies 110 even if the animal moves its leg. Therefore, it is possible to analyze the body composition of the animal simply and accurately.

However, the prior art does not completely limit the movement of the animal. In addition, since the test must be performed while the animal is frightened, there is a defect in that the test result is not accurate. In the case of the biometric information such as the amount of body water, bone density, protein, muscle mass, fat, visceral fat, and basal metabolic rate of the companion animals such as dogs and cats, the impedance changes when muscle movement occurs, resulting in measurement errors, and posture maintenance is required for a certain period of time.

Therefore, in order to accurately measure the biometric information of the companion animal, a means for guiding the companion animal to the measurement position and maintaining the companion animal in a calm state at the measurement position for a certain period of time is required.

### Summary of Invention

### Technical Problem

The present disclosure has been made in an effort to solve the problems described above, and an objective of the present disclosure is to enable exact measurement of biometric information by inducing a companion animal to naturally move to a target area and then maintain a fixed posture.

### Solution to Problem

According to one aspect of the present invention so as to accomplish these objects, there is provided to a system for measuring biometric information of a companion animal, the system including: a body unit having a plate unit on which a companion animal steps; an induction unit installed on a side of the plate unit and inducing a companion animal to maintain a posture in a pre-defined target area for a predetermined time; a plurality of electrode units installed on the plate unit; and a processor unit measuring biometric information of the companion animal on the basis of output signals of the electrodes.

The induction unit may be formed in a shape that is recognized as a closed curve shape by companion animals.

In addition, the induction unit outputs a stimulation signal inducing the companion animal to maintain a posture for a predetermined time and the stimulation signal may include at least any one of a light emission signal, a sound output signal, a fragrance output signal, a wind, and discharge of food.

### Advantageous Effects of Invention

According to the present disclosure, there is an effect that since it is possible to measure exact biometric information of a companion animal, it is possible to check the health condition of a companion animal and enable the companion animal to lead a healthy life.

### Brief Description of Drawings

The above and other objects, features and advantages of the present invention will be more apparent from the following detailed description taken in conjunction with the accompanying drawings, in which:
FIG. 1 illustrates a conventional animal body composition analyzing apparatus;
FIG. 2 is a view showing a schematic structure of a system for measuring biometric information of a companion animal according to a first embodiment of the present disclosure;
FIG. 3 shows an example where an induction unit is discriminated by a color on a plate unit;
FIG. 4 shows an example where the induction unit is 3-dimensionally formed on the plate unit;
FIG. 5 shows an example where the induction unit is implemented as a light emission unit;
FIG. 6 is a view showing the state in which the biometric information of a cat on the induction unit is measured;
FIG. 7 is a block diagram for describing driving and control operation of the position induction device for companion animals according to the present disclosure;
FIG. 8 exemplifies an induction unit having a size variation structure;
FIG. 9 is a view showing the state in which biometric information is measured with a cat lying in the induction unit having a large size;
FIGS. 10 and 11 are views showing schematic structure of a system for measuring biometric information of a companion animal according to a second embodiment of the present disclosure; and
FIG. 12 is a block diagram showing the configuration of an induction unit of the second embodiment of the present disclosure.

### Best Mode

### Mode for Invention

Since the description of the present invention is a mere embodiment for structural and functional description, it must not be interpreted that the scope of the present invention is limited by the embodiments described in the text. That is, since the embodiments can be variously changed and have various forms, it should be understood that the scope of the invention includes the equivalents for realizing the technical concept. Also, since the specific embodiments do not include all objects and effects presented by the present invention, the scope of the present invention is not limited by them.

Unless differently defined, all the terms used here including technical or scientific terms have the same meaning with what is generally understood by one who has common knowledge in the technical field that this invention belongs to. The terms such as those defined in the dictionary commonly used will be interpreted to have the meanings matching with the meanings in the context of the related technologies. Unless clearly defined in this application, they are not interpreted as ideal or excessively formal meanings.

Hereafter, the preferred embodiment according to the present disclosure are described in detail with reference to the accompanying drawings.

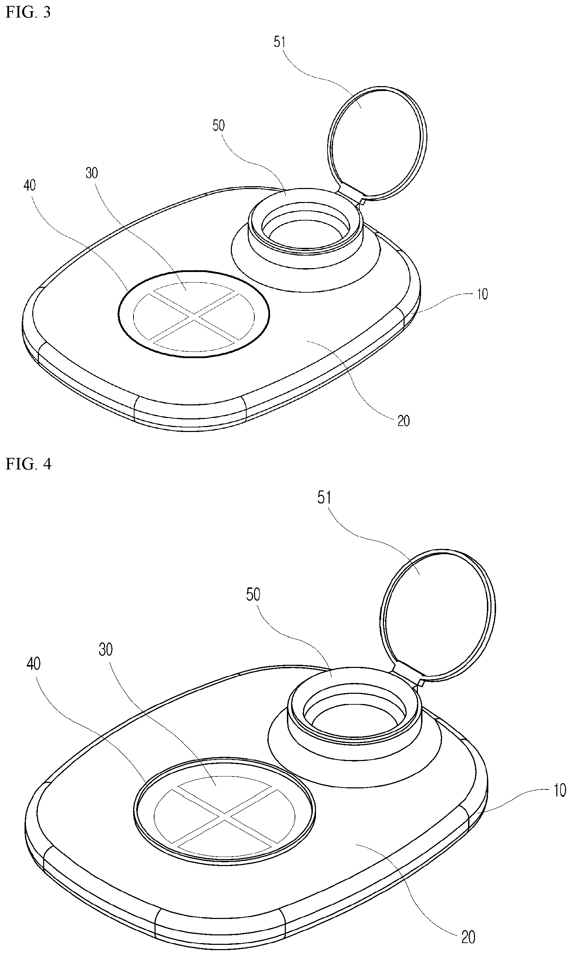

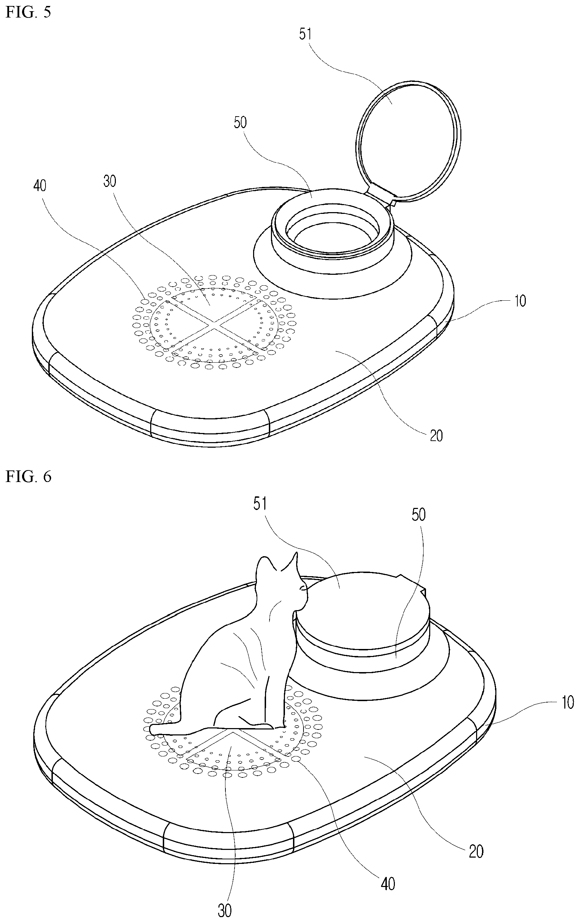

FIG. 2 is a view showing a schematic structure of a system for measuring biometric information of a companion animal according to a first embodiment of the present disclosure, FIG. 3 shows an example where an induction unit is discriminated by a color on a plate unit, FIG. 4 shows an example where the induction unit is 3-dimensionally formed on the plate unit, FIG. 5 shows an example where the induction unit is implemented as a light emission unit, and FIG. 6 is a view showing the state in which the biometric information of a cat on the induction unit is measured.

As shown in FIG. 2, in a system for measuring biometric information of a companion animal according to a first embodiment of the present disclosure, a plate unit 20 on which a companion animal steps is formed on the top of a body unit 10, and an electrode unit 30 for measuring biometric information of a companion animal, an induction unit 40 formed in a closed curve shape to surround the electrode unit 30 and inducting a companion animal into the closed curve, and a feeder 50 are installed on the plate unit 20.

The electrode unit 30 has four electrodes spaced apart from each other to be able to apply a current to the soles of four-legged animals, respectively. The electrodes may be manufactured in various shapes, sizes, and disposition structures depending on the kinds or sizes of target companion animals, and it is preferable that the electrodes are formed at the positions of four feet when a companion animal sits.

As for the biometric information body composition of the trunk of a companion animal, a current is applied to flow from the right front foot to the right rear foot, a voltage is measured from the left front foot to the left rear foot, and a resistance value is measured at the middle overlap portion, whereby it is possible to measure the biometric information body composition of the companion animal. The biometric information that is measured in this case may be various items of information such as the amount or ratio of weight, body fat, lean body mass, muscles, in-body water, bones, or the like, and appropriate biometric measurement devices may be used depending on the items of information.

The induction unit 40 uses the habit of companion animals that they feel stable when being inside a closed curve, and it is formed in a closed curve shape sized such that companion animals can sit therein. In the present disclosure, the term `closed curve', which means the shapes of figures that can be recognized as closed curve shapes by companion animals, is a concept including various shapes of figures entirely showing a closed curve shape such as a closed curve of a dotted line, a closed curve of a solid line, and partially disconnected closed curve.

Further, the closed curve may have the shapes of various figures such as a circle, an ellipse, a rectangle, a triangle, and a pentagon, a circle being the most general shape is exemplified in this embodiment, and the following description exemplifies cases where all the closed curve shapes are circles.

The induction unit 40 may be various devices that enable companion animals to recognize the circle on the plate unit 20.

For example, the induction unit 40 shown in FIG. 3 may be a circle of a thick solid line different from the background color of the plate unit 20. Alternatively, the induction unit 40, as shown in FIG. 4, may be formed like a loop structure protruding upward so that companion animals can more easily recognize the shape of the circle.

As another embodiment, as shown in FIG. 5, the induction unit 40 may be installed in a form employing a light emission device, such as an LED, that is disposed in a circular shape and turns on/off or a form employing a projector that emits circular image light. In the following description, a case in which several LED light sources, which can most effectively show the position induction unit 40 and can show a circle while changing the position and size of the circle, are installed in a circular shape is exemplified.

When several LED light sources are installed in a circular shape, the LED light sources may be arranged in the form of a plurality of coaxial circles such that the diameter of the induction unit 40 can be changed. The induction unit 40 may be shown in a sufficiently large size in the early stage so that companion animals can easily recognize the induction unit 40, and then may be shown in a small size when a predetermined time passes or approach of a companion animal is sensed by a proximity sensor, etc. This is for making companion animals be positioned in a smaller circle with four soles exactly positioned on the electrodes.

Meanwhile, as another embodiment of the induction unit 40, a touch input device such as a touch screen may be disposed on the surface of the plate unit 20 such that when a user draws a specific closed curve shape on the touch input device using a finger, etc., the drawn closed curve shape is shown by a light emission device. As another method, a user terminal may be connected to the body unit 10 through a communication means such that when a user inputs a specific closed curve shape through the user terminal, the closed curve shape is shown by a light emission device.

It may be a little difficult to induce dogs to a desired position using a closed curve shape in comparison to cats. On the other hand, cats are difficult to train whereas dogs are much easier to train. Using this characteristics, a user can train a companion dog for a specific situation of showing a specific closed curve shape on the surface of the plate unit 20, whereby it is possible to induce a trained companion dog to move to a target area having the closed curve shape on the induction unit. For example, it is possible to induce a companion dog to move to the position induction unit by training the companion dog to recognize `positioning in a circle' as eating snacks, `positioning in a rectangle' as eating feed, `positioning in a triangle' as eating special snacks, etc.

The feeder 50 is a device installed on a side of the body unit 10 and providing food such as feed to companion animals, and it is preferable that the feeder 50 is configured to open a dispensing port when a companion animal is positioned in a circle of the induction unit. This is because companion animals easily maintain a sitting posture over a predetermined time while they eat feed in the circle with the dispensing port open. Opening the dispensing port may be implemented in various types such as a type of opening a cover 51 of the feeder 50 and a type of opening the dispensing port in a slide or hinge type.

FIG. 6 shows the case in which a companion animal maintains a posture to eat food in the induction unit 40 with the cover 51 open.

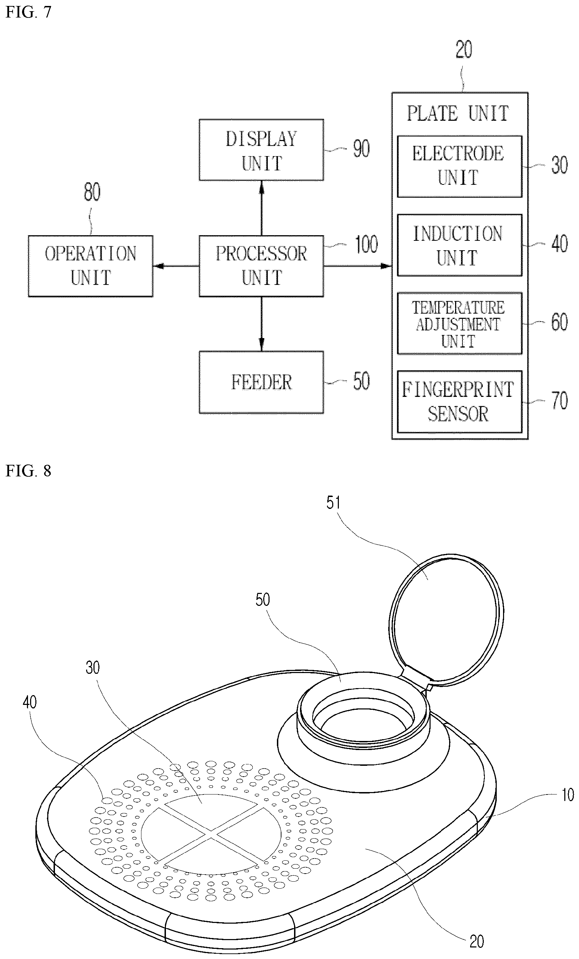

FIG. 7 is a block diagram for describing driving and control operation of the position induction device for companion animals according to the present disclosure.

Referring to FIG. 7, a temperature adjustment unit 60 and a fingerprint sensor 70 may be further provided at the plate unit 20 in addition to the electrode unit 30 and the induction unit 40.

The temperature adjustment unit 60, which is for adjusting the temperature of the plate unit 20, may include a cooler, a heater, or both a cooler and a heater. When a user operates the temperature adjustment unit 60 in accordance with a season, it is possible to make a companion animal stay longer in a target area defined by the induction unit 40 the space inside the induction unit. To this end, it is preferable that the temperature adjustment unit 60 is disposed at a position corresponding to the induction unit 40.

The fingerprint sensor 70, which is disposed on a side of the plate unit 20 to correspond to the induction unit 40 and detects fingerprints of companion animals, may be used as a device for recognizing individual information of companion animals. When a user has a plurality of companion dogs, it is impossible to know which measured biometric information is the biometric information of which companion animal. To this end, in the present disclosure, a fingerprint sensor is installed in the position induction unit 10, whereby it is possible to identify companion animals.

Further, a display unit 90 for displaying the operation state of the system and measured biometric information and an operation unit 80 for operating the system may be disposed on the body unit 10.

Further, a processor unit 100 for controlling driving of the system is disposed in the body unit 10. The processor unit 100 performs various control operations and calculation operations such as controlling turning-on/off of the induction unit 40, analyzing fingerprints detected by the fingerprint sensor 70, identifying individual companion animals, adjusting the temperature of the plate unit 20 on the basis of operation by a user, applying electricity to the electrode unit 30, measuring biometric information, and controlling driving of the display unit 90.

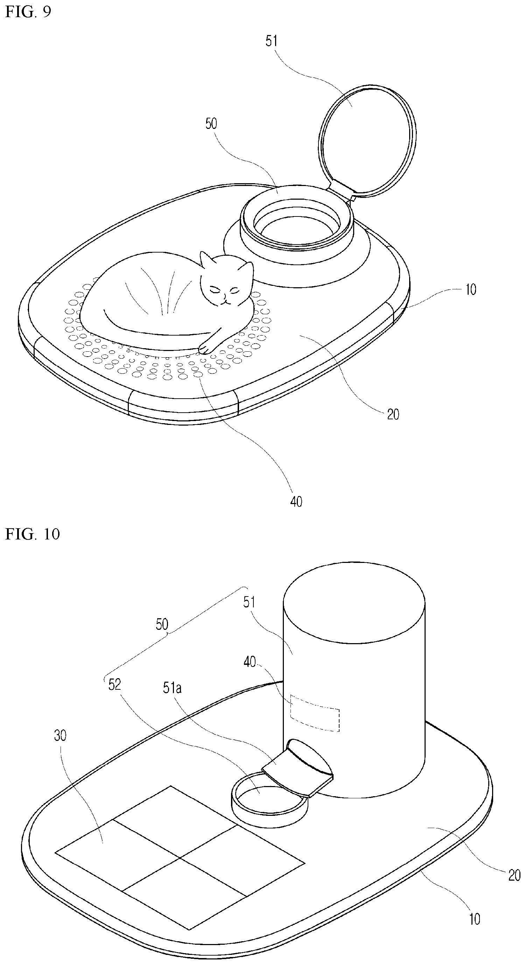

FIG. 8 exemplifies an induction unit having a size variation structure, and FIG. 9 is a view showing the state in which biometric information is measured with a cat lying in the induction unit having a large size.

As shown in FIG. 8, when the induction unit 40 is a light emission device, it can be turned on and off to change the size of the circle. In this figure, a circle having a larger diameter than the induction unit 40 of FIG. 5 is shown. As for the size of the induction unit 40, an appropriate size can be selected in accordance with the size of a companion animal or the circle may be set in different sizes in accordance with the types of biometric information to be measured.

For example, in order to measure general biometric information such as a weight or a body composition, it is possible to measure biometric information of a desired companion animal with the induction unit 40 having the size of FIG. 5 shown. Meanwhile, in order to measure a life pattern such as the amount of sleep or the amount of rest of a companion animal, a larger circle may be shown, as shown in FIG. 8. Companion animals may maintain a sitting posture in a small circle and maintain a comfortably lying posture in a large circle in many cases. Accordingly, in order to measure a life pattern of a companion animal, it is possible induce a companion animal to comfortably lie inside the induction unit 40, as shown in FIG. 9, by maintaining a preferred temperature of the companion animal through the temperature adjustment unit 60 with a large circle turned on the plate unit 20.

As described above, it is possible to not only measure biometric information of a companion animal, but provide a space in which a companion animal can relax through the combination of the induction unit 40 and the temperature adjustment unit 60.

### Description of Embodiments

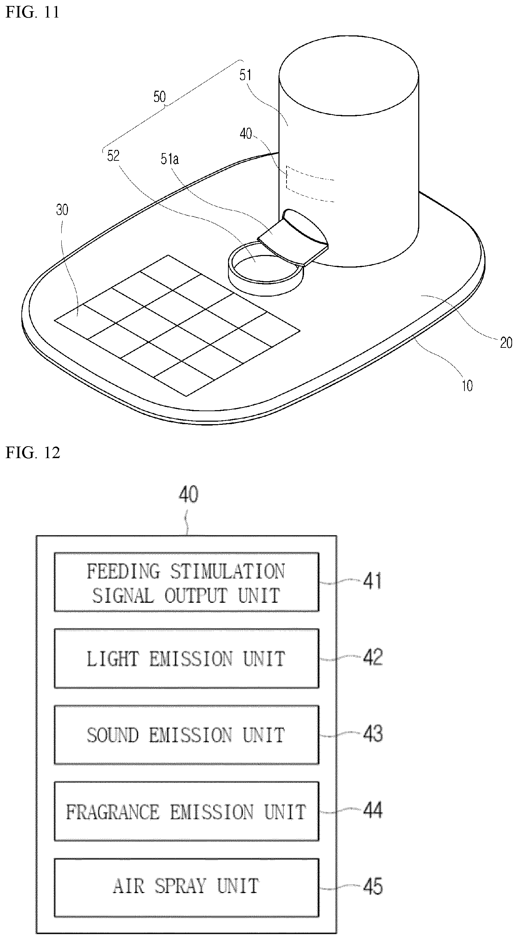

FIGS. 10 and 11 are views showing schematic structure of a system for measuring biometric information of a companion animal according to a second embodiment of the present disclosure and FIG. 12 is a block diagram showing the configuration of an induction unit of the second embodiment of the present disclosure.

As shown in FIGS. 10 and 11, in a system for measuring biometric information of a companion animal according to a first embodiment of the present disclosure, a plate unit 20 on which a companion animal steps is formed on the top of a body unit 10, and electrode units 30 for measuring biometric information of a companion animal, an induction unit 40 inducing a companion animal to maintain a posture for a predetermined time, and a feeder 50 are installed on the plate unit 20.

The electrode units 30 are arranged in blocks in a lattice shape to be able to apply a current to the soles of four-legged animals. This is for solving the problem that it is difficult to make companion animals place their feet in a specific area of the plate unit 20 in order to measure biometric information. In detail, a controller 60 detects electrodes, on which the feet of a companion animal are positioned, of the electrode unit 30, and measures impedance through the detected electrodes, whereby it is possible to easily measure biometric information of the companion animal.

The electrodes may be manufactured in various shapes, sizes, and disposition structures depending on the kinds or sizes of target companion animals. For example, as shown in FIG. 10, as for medium-size and large-size dogs, the electrode units 30 may be formed in a lattice shape with four sections having a large area to detect two front feet and measure biometric information. Further, as for small-size dogs, as shown in FIG. 11, electrodes are formed in a lattice shape with more blocks to detect all of the four legs of a companion animal.

The induction unit of the second embodiment is installed at a side of the feeder 50 and outputs a stimulation signal, and various devices that generate a stimulation signal may be used individually or in a combination. The stimulation signal is a means that induce a companion animal to expect that food will be discharged soon from a dispensing port 51a and stand by in a fixed posture in front of a food container 52. It is possible to make companion animal expect discharge of food through training, and general companion animals can sufficiently learn that food will be provided within a predetermined time after a stimulation signal is generated through one-time or several-time training. In particular, when discharge of a small amount of food is used as the stimulation signal, it is possible to induce companion animal to stand by in a fixed posture in front of the food container 52 through an instinctive reaction even without a specific training process.

As an example, it is possible to include a light emission unit 41 that emits light, a sound output unit 42 that outputs a specific sound, a fragrance emission unit 43 that emits specific fragrance, an air spray unit 44 that generates wind by spraying air, etc., in which a light emission signal, a sound signal, a fragrance output signal, a wind spray signal, operation of opening/closing a dispensing port, etc. may be stimulation signals.

The feeder 50, which is a device installed on a side of the body unit 10 and providing food such as feed to companion animals, discharges a small amount of food when the dispensing port 51a is opened in response to a stimulation signal.

In the second embodiment of the present disclosure, it is possible to use the signal that is output from the induction unit as both a position induction signal for inducing a companion animal to move to a measurement position and a posture maintenance induction signal for inducing the companion animal to maintain a posture at the measurement position. For example, it is possible to attract companion animal's attention and induce the companion animals to move to a target area by providing signals such as a light emission signal, a sound output signal, a fragrance output signal, blowing a wind, and discharging a small amount of food.

Thereafter, an example of using discharge of a small amount of food as an output signal of an induction unit is described.

The processor unit 100 controls the feeder 50 to discharge a small amount of food. When a small amount of food is discharged, a companion animal steps on the plate unit 20 to eat the food and then eat the food discharged from the food container 52.

The processor unit 100 specifies electrodes on which the soles of the companion animal are positioned by detecting variation of impedance of the electrode unit 30, and determines the electrodes as electrodes for measuring biometric information.

The processor unit 100 repeats the control for discharging a small amount of food after a predetermined time passes, and gradually increases the intervals of the actions of discharging food. Accordingly, the companion animal maintains a fixed posture while expecting the food will keep being provided.

The processor unit 100 repeatedly measure biometric information while the companion animal stands by while discharging food for a predetermined number of times, and stops discharging food when a predetermined number of times of measuring is finished.

### Industrial Applicability

The present invention is a very useful invention in the industry because it is possible to easily and accurately measure the biometric information of the companion animals.

## Claims

1. A system for measuring biometric information of a companion animal, the system comprising:
a body unit having a plate unit on which a companion animal steps;
an induction unit installed on a side of the plate unit and inducing a companion animal to maintain a posture in a pre-defined target area for a predetermined time;
a plurality of electrode units installed on the plate unit; and
a processor unit measuring biometric information of the companion animal on the basis of output signals of the electrodes.

2. The system of claim 1, wherein the induction unit is formed in a shape that is recognized as a closed curve shape by companion animals.

3. The system of claim 2, wherein the closed curve shape is implemented by any one method of a color, a protruding shape, and a light emission device.

4. The system of claim 3, wherein a light emission position and a size of the light emission device are changed, or the position and the size are simultaneously changed.

5. The system of claim 1, wherein a temperature adjustment unit adjusting temperature of the plate unit is installed on a side of the body unit.

6. The system of claim 1, further comprising a fingerprint sensor disposed on a side of the plate unit to correspond to the position induction unit, and detecting a fingerprint of the companion animal,
wherein individual information of the companion animal is identified on the basis of fingerprint information detected by the fingerprint sensor.

7. The system of claim 1, wherein the induction unit outputs a stimulation signal inducing the companion animal to maintain a posture for a predetermined time.

8. The system of claim 7, wherein the stimulation signal includes at least any one of a light emission signal, a sound output signal, a fragrance output signal, a wind, and discharge of food.

9. The system of claim 8, wherein when the processor unit recognizes electrodes on which feet of a companion animal are positioned, the processor unit control the stimulation signal to be output and measures biometric information of the companion animal at a set point in time after the stimulation signal is output.

10. The system of claim 7, wherein the processor unit outputs the stimulation signal a plurality of times and controls intervals of output times for outputting the stimulation signal.

11. The system of claim 10, wherein the processor unit outputs the stimulation signal a plurality of times and increases the intervals of the output times for outputting the stimulation signal.
